(19) Europäisches Patentamt
European Patent Office
Office européen des brevets

(11) Veröffentlichungsnummer: **0 149 634**
**B1**

(12) # EUROPÄISCHE PATENTSCHRIFT

(45) Veröffentlichungstag der Patentschrift: **12.10.88**

(51) Int. Cl.⁴: **C 12 N 15/00**, C 07 H 21/04

(21) Anmeldenummer: **84902585.3**

(22) Anmeldetag: **28.06.84**

(86) Internationale Anmeldenummer: **PCT/EP 84/00196**

(87) Internationale Veröffentlichungsnummer: **WO 85/00621 (14.02.85 Gazette 85/04)**

(54) **VERFAHREN ZUR DEFINIERTEN VERKNÜPFUNG DOPPELHELIKALER DNA-FRAGMENTE MITTELS EINER LINKER-DNA SOWIE ALS LINKER-DNA GEEINGNETE OLIGONUCLEOTIDE.**

(30) Priorität: **22.07.83 DE 3326520**

(43) Veröffentlichungstag der Anmeldung: **31.07.85 Patentblatt 85/31**

(45) Bekanntmachung des Hinweises auf die Patenterteilung: **12.10.88 Patentblatt 88/41**

(84) Benannte Vertragsstaaten: **AT BE CH DE FR GB LI LU NL SE**

(56) Entgegenhaltungen:
**EP-A-0 036 258**
**EP-A-0 046 669**
**GB-A-2 073 245**
**US-A-4 321 365**

(73) Patentinhaber: **BIOSYNTECH Biochemische Synthesetechnik GmbH, Stresemannstrasse 268-280, D-2000 Hamburg 50 (DE)**

(72) Erfinder: **KÖSTER, Hubert, Hallerstrasse 74, D-2000 Hamburg 13 (DE)**

(74) Vertreter: **Henkel, Feiler, Hänzel & Partner, Möhlstrasse 37, D-8000 München 80 (DE)**

**EP 0 149 634 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft ein Verfahren zur definierten Verknüpfung doppelhelikaler DNA-Fragmente über kohäsive Enden mit den im Anspruch 1 angegebenen Merkmalen. Die Erfindung betrifft weiterhin eine doppelhelikale Linker-DNA der im Anspruch 1 angegebenen Formel I, in der $R^1$, $R^2$, X und n wie oben definiert sind. Das Verfahren bzw. die Linker-DNA der Erfindung kann für eine gezielte Veränderung des Erbmaterials verwendet werden.

Restriktions-Linker sind bekannt (Scheller et al., Science Bd. 196, (1977), 177 und werden seit langem in der Molekularbiologie und Gentechnologie verwendet. Ihnen ist gemeinsam, daß die Erkennungsregion für die Restriktionsendonuclease im zentralen Teil der Oligonucleotidsequenz liegt und in der Regel im 5' - und 3'-endständigen Bereich von G-/C-reichen Sequenzen flankiert wird. Im folgenden werden zwei Beispiele für bekannte Linker-Sequenzen gegeben:

| | |
|---|---|
| Eco RI-Linker: | d(GGAATTCC) |
| Bam HI-Linker: | d(CGGATCCG) |

Die Nachteile dieser Linker sollen im folgenden an einem Beispiel erläutert werden. Nach Ligation dieser Linker an DNA mit Hilfe von T4-Polynucleotidligase wird durch diese Linker das Erbmaterial (DNA) unweigerlich und irreversibel verändert, da nach dem "Schneiden" mit der entsprechenden Restriktionsendonuclease (gegebenenfalls nach erfolgter Klonierung etc.) auch nach Anwenden einer einzelstrangspezifischen Endo-/Exonuclease, ein Teil der angekoppelten Linkernucleotide an der DNA verbleiben:

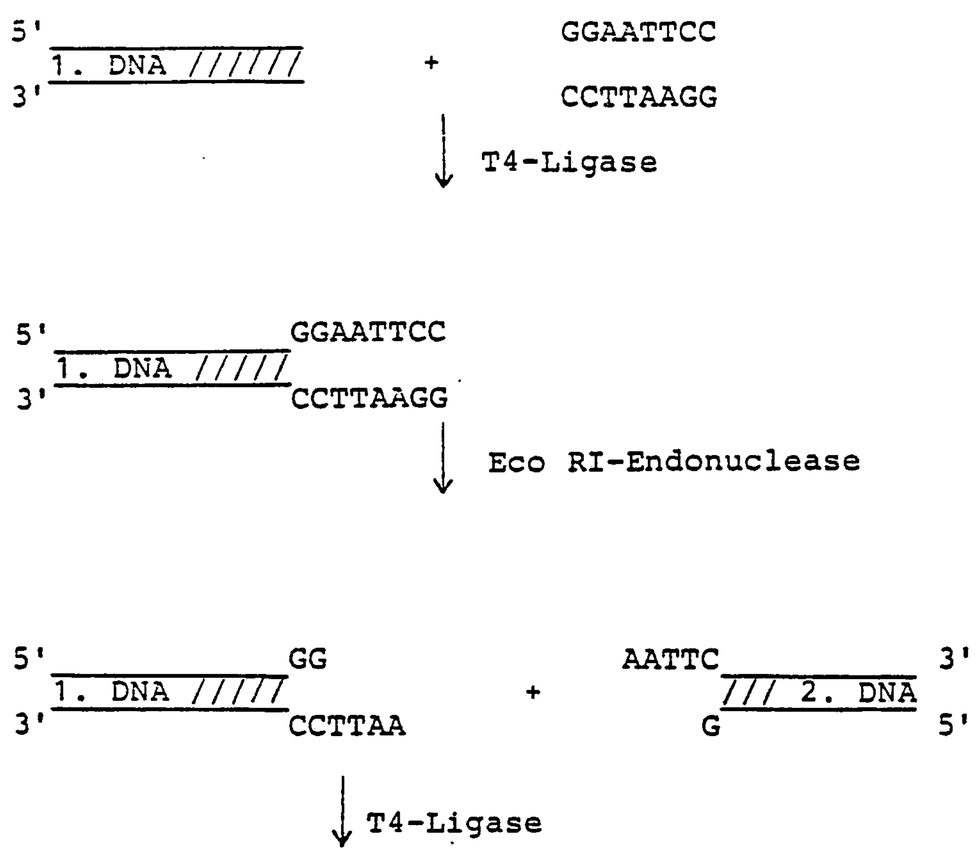

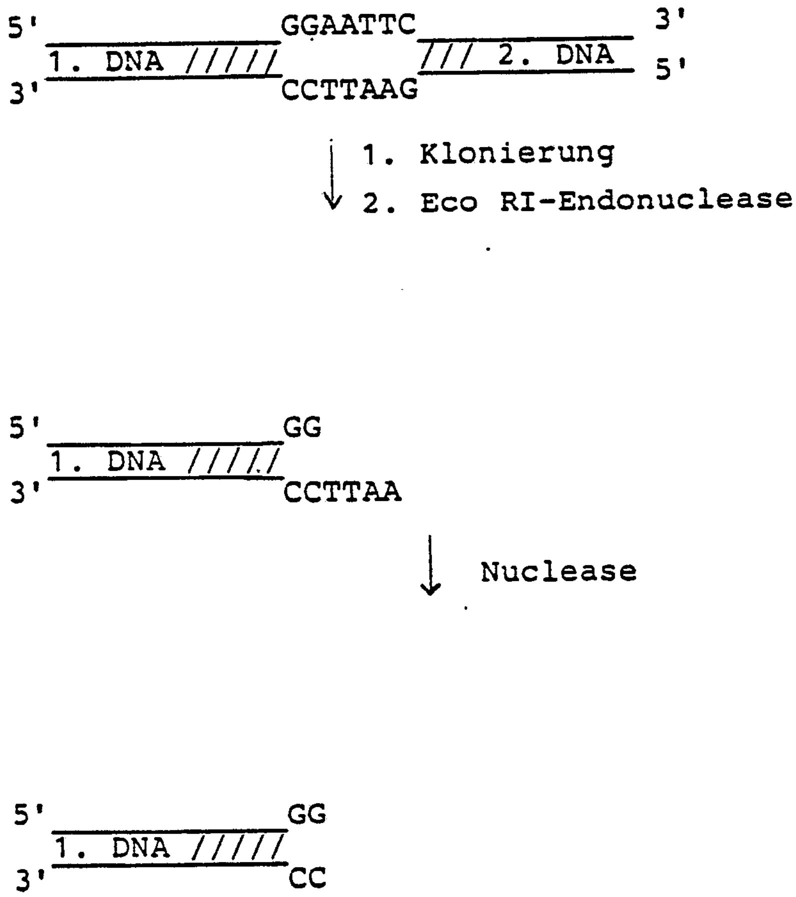


Es wird deutlich, daß bei diesem Beispiel gemäß dem Stand der Technik die ursprüngliche DNA um 2 GC-Basenpaare verlängert wurde.

Die Nachteile des bekannten Verfahrens können mit dem Verfahren bzw. der Linker-DNA der Erfindung vermieden werden, da sie ermöglichen, die ursprüngliche Erbinformation in unveränderter Weise zurückzugewinnen. Die Merkmale des Verfahrens der Erfindung ergeben sich aus dem kennzeichnenden Teil des Anspruchs 1.

Der hier verwendete Begriff "komplementär" bedeutet komplementär im Sinne von Watson und Crick. "Spiegelbildlich komplementär" bedeutet, daß zwei Sequenzen komplementär sind, wenn die eine von links nach rechts und die andere von rechts nach links gelesen wird.

Die Oligonucleotide 1 weisen normalerweise ebenso wie die ersten und zweiten DNA-Fragmente am 5'-Ende eine Phosphatgruppe auf; fehlt diese am Oligonucleotid I, ist lediglich dafür zu sorgen, daß das 1. bzw. 2. DNA-Fragment an der Verknüpfungsstelle für die Linker-DNA eine 5'-ständige Phosphatgruppe aufweist. Fehlt die Phosphatgruppe im ersten oder zweiten DNA-Fragment, muß das Oligonucleotid I eine 5'-Phosphatgruppe aufweisen.

Wenn $R^1$ eine der durch Kombination der vier Nucleotide A, T, C und G möglichen, nicht in sich selbst

komplementären Tetranucleotidsequenzen ist, ergeben sich die aus Tabelle 1 ersichtlichen Sequenzen. Wenn $R^1$ eine der durch Kombination der vier Nucleotide A, T, C und G möglichen, nicht in sich selbst komplementären Pentanucleotide ist (in Anbetracht der ungeraden Nucleotidzahl sind Pentanucleotidsequenzen ausschließlich nicht in sich selbst komplementär), erhält man durch analoge Permutation die hier im einzelnen nicht wiedergegebenen möglichen Sequenzen, z. B.

(5')AAAAA(3')
AAAAT
.
.
.
.
.
.
GGGGC
GGGGG

Sinngemäß können auch durch entsprechende Permutation die Kombinationsmöglichkeiten für in sich selbst oder nicht in sich selbt komplementäre Hexanucleotide aus den Nucleotiden A, T, C und G erhalten werden, z. B.

(5')AAAAAA(3')
(5')AAAAAT(3')
AAAATT
AATTTT
.
.
.
.
.
.
GGCCCC
GGGGCC
GGGGGC
GGGGGG

Für die hier angesprochenen Fälle, daß $R^1$ eine nicht in sich selbst komplementäre Tetra- oder Penta- oder eine in sich oder nicht in sich selbst komplementäre Hexanucleotidsequenz ist, gilt, daß $R^2$ die zu $R^1$ spiegelbildlich komplementäre Sequenz mit gleicher Kettenlänge aufweissen muß, z. B.

(5')ACTA$(X)_n$TAGT(3')
(5')CACACA$(X)_n$TGTGTG(3')
(5')AAATTT$(X)_n$AAATTT(3')

Weiterhin gilt für diese Fälle, daß entweder $R^1$ oder $R^2$ ein einziges Ribonucleotid enthalten muß, und zwar in den endständigen Positionen 3' oder 5', oder gegebenenfalls auch X ein 3'- oder 5'-endständiges Ribonucleotid enthält, z. B.

(5')C*ACAGC*TGTG(3'),

wobei $R^1$ = C*ACA, $R^2$ = TGTG und X = GC* bedeuten und die Ribonucleotide durch * markiert sind.

Es gilt die Regel, daß zur Erzeugung kohäsiver Enden in dem Spacer X ein Ribonucleotid vorhanden sein muß, wenn entweder $R^1$ oder $R^2$ ein Ribonucleotid aufweisen; ein Ribonucleotid im Spacer X ist dagegen nicht erforderlich, wenn $R^1$ und $R^2$ je ein Ribonucleotid enthalten.

Es ist jedoch auch möglich, daß $R^1$ und $R^2$ 5'- oder 3'-ständige Ribonucleotide enthalten, wobei die jeweilige Positionierung des Ribonucleotids bestimmt, welche Kettenlänge das übertragene kohäsive Ende aufweist.

Weiterhin gilt, daß T* (Ribothymidin) durch das gleichwirkende U* (Ribouridin) ersetzt sein kann.

Eine Aufzählung und Beschreibung von Restriktionsendonucleasen der Klassen II und III ist in der Firmenschrift "Restriction Endonucleases" der Boehringer Mannheim GmbH, 1981, enthalten; diese Veröffentlichung enthält auch Restriktionsendonucleasen der Klassen II und III, die für das Verfahren der Erfindung verwendbar sind, in der folgenden Beschreibung jedoch nicht namentlich genannt werden.

Mit dem Verfahren der Erfindung lassen sich 1, 2, 3, 4, 5 oder 6, vorzugsweise 4, Nucleotide aufweisende kohäsive Enden auf die erste DNA übertragen, wobei Nucleotide aus der Spacer-Gruppe X nicht mit übertragen werden. Es ist jedoch auch möglich, wie später an einem Beispiel gezeigt wird, aus dem Spacer X ein oder mehrere Nucleotide mit auf die erste DNA zu übertragen, und zwar bei geeigneter Positionierung

eines Ribonucleotids in der Gruppe X.

Die Ausbildung der kohäsiven Enden an der 1. DNA erfolgt durch Spaltung des aus der 1. DNA und der Linker-DNA erhaltenen Reaktionsproduktes, das in allgemeiner Schreibweise wie im Schema I dargestellt werden kann (am Schluß der Beschreibung).

Im Schema 1 bedeutet N die die Reste $R^1$ bildenden Nucleotide A, T, G und/oder C bzw. A*, T*, G*, C* und/oder U*, und X die oben definierte Oligonucleotidgruppierung mit 2 - 30 Nucleotideinheiten. Weiterhin sind mit Pfeilen die Spaltungspositionen gemäß vier Spaltungsvarianten a, b, c und d dargestellt, auf die im folgenden eingegangen wird.

**Varianten a, b:**

Die Spaltung erfolgt mittels Restriktionsendonucleasen der Klassen II bzw. III und führt zu der Ausbildung eines kohäsiven Endes an der Fremd-DNA, das die gleiche Nucleotidzahl aufweist wie die Gruppe $R^1$ bzw. $R^2$ der Linker-DNA. Je nach Spezifität der Endonuclease wird das kohäsive Ende am 5-' oder 3'-Ende der Fremd-DNA erzeugt.

**Varianten c, d:**

Die Spaltung erfolgt mittels Basen bzw. RNA-sen, setzt somit voraus, daß an den Spaltungspositionen Ribonucleotide A*, T*, C*, G* oder U* vorhanden sind, wobei zu beachten ist, daß die Spaltung immer am 3'-Ende des Ribonucleotids erfolgt. Je nach Positionierung der Ribonucleotide wird das kohäsive Ende am 5'- oder 3'-Ende der Fremd-DNA erzeugt; weiterhin bestimmt die Positionierung der Ribonucleotide die Kettenlänge (1 - 6) des übertragenen kohäsiven Endes. Das kohäsive Ende kann ausschließlich aus den Nucleotiden der Gruppen $R^1$ und $R^2$ stammen; es können jedoch zusätzlich noch Nucleotide aus der Spacerregion übertragen werden.

Gemäß einer weiteren vorteilhaften Ausführungsform der Erfindung kann man das ein kohäsives Ende aufweisende, erste DNA-Fragment vor der Verknüpfung mit dem zweiten DNA-Fragment mit einer an sich bekannten, kohäsive Enden unterschiedlicher Restriktionsspezifizität aufweisenden Adapter-DNA umsetzen. Auch für diese Variante wird später noch ein Beispiel gegeben.

Das Verfahren der Erfindung wird im folgenden anhand von bevorzugten Beispielen näher erläutert.

Wenn in den Oligonucleotiden der allgemeinen Formel I $R^1$ und $R^2$ Sequenzen der allgemeinen Formel II bedeuten, d.h. z. B. die zentrale Erkennungsregion von Restriktionsendonucleasen der Klassen II und III, und $(X)_n$ eine kurze Spacerregion darstellt, lassen sich mit diesen Oligonucleotiden mit Hilfe von Ligasen, insbesondere $T_4$-Polynucleotidligase Erkennungsregionen für Restriktionsendonucleasen in genetisches Material (DNA) einbauen. Dabei gestatten es die Oligonucleotide der Formel I, daß nach erfolgter Kopplung und Klonierung die ursprüngliche Erbinformation in unveränderter Form zurückgewonnen wird. Dies wird dadurch erreicht, daß sich die den kohäsiven Teil beinhaltende Signalsequenz der Restriktionsendonuclease an den beiden Enden des Oligonucleotids befindet.

Das folgende Beispiel 1 verdeutlicht die Vorteile, die mit einem Oligonucleotid der Erfindung der Formel d-(AATTGGAATT) erreichbar sind.

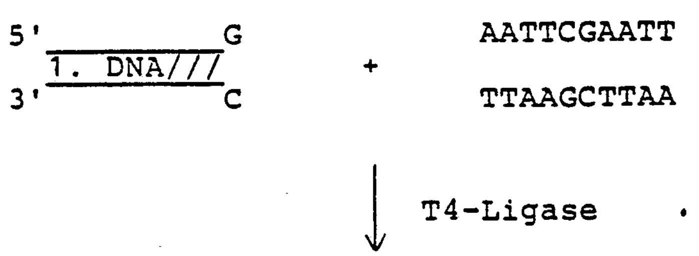

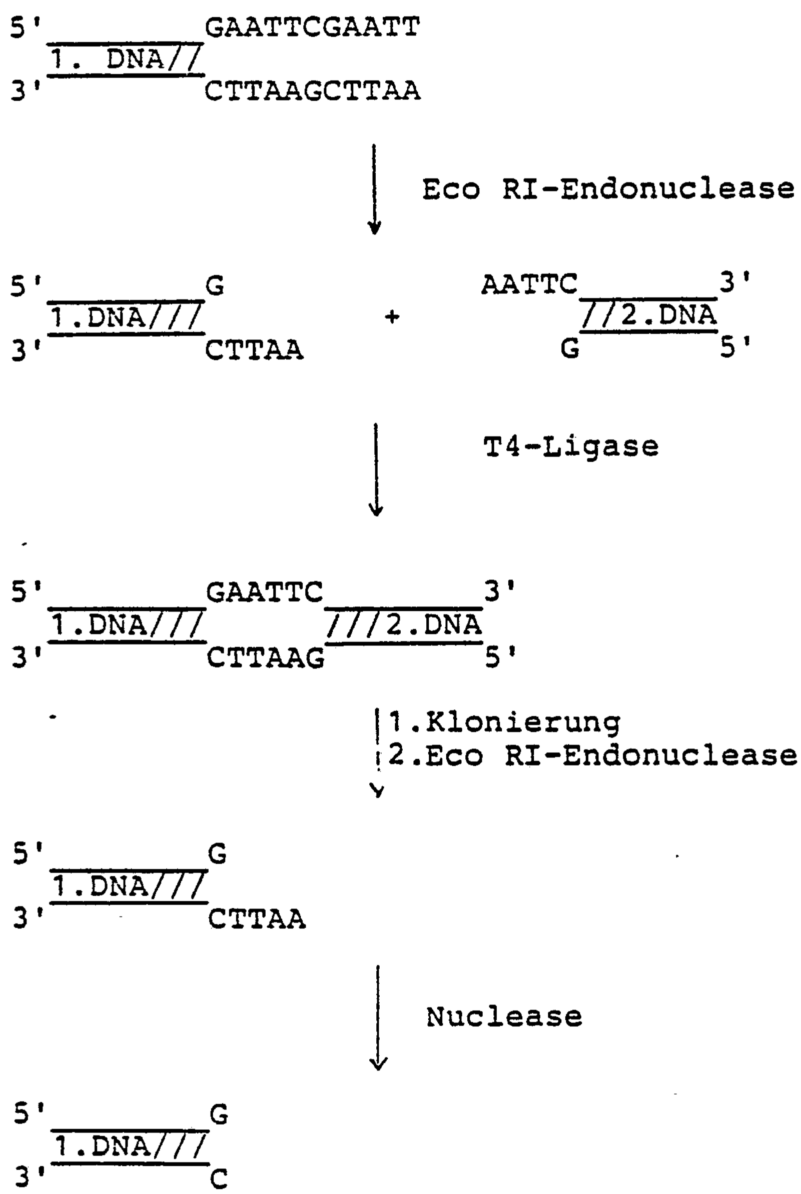


Es wird deutlich, daß mit dem Verfahren bzw. dem Oligonucleotid der Erfindung die Erbinformation (1. DNA) nach Kopplung derselben mit dem Oligonucleotid der Erfindung bzw. der Linker-DNA, Spaltung unter Erzeugung eines kohäsiven Endes, Kopplung an eine zweite DNA (Vektor-DNA) mit einem komplementären kohäsiven Ende, Klonieren, erneute Spaltung und Abspaltung des kohäsiven Endes in unveränderter Form zurückerhalten wird.

Die Einführung Endonuclease-spezifischer kohäsiver Enden gemäß der Erfindung hängt vom endständigen Basenpaar der ersten DNA ab. Einige Beispiele für die vier möglichen Basenkonstellationen sollen das Verfahren der Erfindung weiterhin erläutern:

**Beispiel 2**

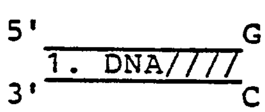


| | |
|---|---|
| Oligonucleotide I: | AATTCGAATT (Signalsequenz für Eco RI) |
| | GATCCGGATC (Signalsequenz für Bam HI) |

GTACCGGTAC (Signalsequenz für Kpn I)
TCGACGTCGA (Signalsequenz für Sal I)
AGCTCGAGCT (Signalsequenz für Sac I/Sst I)

**Beispiel 3**

```
5' ___________ A
  1. DNA////
3' ___________ T
```

Oligonucleotide I: GATCTAGATC (Signalsequenz für Bgl II)
TCGATATCGA (Signalsequenz für Cla I)
AGCTTAAGCT (Signalsequenz für Hind III)
AATTTAAATT (Signalsequenz für Aha III)

**Beispiel 4**

```
5' ___________ C
  1. DNA////
3' ___________ G
```

Oligonucleotide I: TGCAGCTGCA (Signalsequenz für Pst I)
GATCGCGATC (Signalsequenz für Pvn I)
TCGAGCTCGA (Signalsequenz für Xho I)

**Beispiel 5**

```
5' ___________ T
  1. DNA////
3' ___________ A
```

Oligonucleotide I: GATCATGATC (Signalsequenz für Bcl I)
GTAGATCTAG (Signalsequenz für Xba I)

Für die Beispiele 1 - 5 ist die Kenntnis des endständigen Basenpaares der ersten DNA (Fremd-DNA) erforderlich. Demgegenüber genügt für einige Restriktionsendonucleasen das Vorhandensein der selbst komplementären Tetranucleotidsequenz, so daß diese Linker gemäß dem Verfahren der Erfindung für jede Fremd-DNA verwendet werden können, gleichgültig, welches endständige Basenpaar dort vorliegt, d.h. nach Ankopplung einiger der Oligonucleotide der Erfindung wird immer das kohäsive Ende der entsprechenden Restriktions endonuclease erzeugt. Im folgenden werden hierfür einige Beispiele gegeben.

**Beispiel 6**

```
5'_____________N
  1. DNA/////
3'_____________N'
```

Oligonucleotide I: GATCCGGATC (Signalsequenz für Mbol /Sau 3 A)
TCGAGCTCGA (Signalsequenz für Taq I)
AATTCGAATT (Signalsequenz für Eco RI*)
AGCTCGAGCT (Signalsequenz für Alu I)

N ist eines der Nucleotide A, C, G und T; N' bedeutet das jeweils dazu komplementäre Nucleotid A, C, G und T.

Im folgenden wird ein Beispiel für den Einsatz eines Oligonucleotids gegeben, in dem $R^1$ eine Sequenz der Formel III und $R^2$ eine Sequenz der Formel IV mit den dort angegebenen Definitionen für A, C, G und T bzw. A*, C*, G* und T* sowie N und N' bedeuten, wobei die Sequenzen von $R^1$ und $R^2$, von links nach rechts gelesen, bis auf die zueinander komplementären Nucleotide N und N', identisch sind.

**Beispiel 7**

```
5'_____________G
  1. DNA/////
3'_____________C
```

Oligonucleotide I: GTCACCGGTGAC (Signalsequenz für Bst EII)

Falls aufgrund des gegebenen endständigen Basenpaares der Fremd-DNA nicht direkt die gewünschten kohäsiven Enden erzeugt werden können, gelingt dies in einem zwischen den Spaltungs- und Verknüpfungsschritt eingeschalteten zusätzlichen Verfahrensschritt. Im nachfolgenden Beispiel 8 besitzt die erste DNA (Fremd-DNA) ein endständiges GC-Basenpaar, so daß die für Hind III spezifischen kohäsiven Enden nicht direkt eingeführt werden können. Es wird daher zunächst der Bam HI-Linker und danach eine Bam HI-/Hind III-Adapter-DNA verwendet, um das gewünschte Ziel zu erreichen (Adapter-DNA kann als kurze DNA mit kohäsiven Enden unterschiedlicher Restriktionsspezifität definiert werden):

**Beispiel 8**

```
5'____________G          GATCCGGATC
  1. DNA////        +
3'____________C          CTAGGCCTAG

                 |
                 | 1.T4-Ligase
                 | 2.Bam HI - Endonuclease
                 v

 5'____________G                  GATCCCGGGTA
   1. DNA////.              +
 3'____________CCTAG                  GGCCCATTCGA

                 |
                 | T4-Ligase
                 v

 5'____________G GATCCCGGGTA
   1. DNA////
 3'____________CCTAG GGCCCATTCGA
```

Eine Verfahrensführung gemäß Beispiel 8 ermöglicht letztlich die Einführung jedes kohäsiven Endes unabhängig davon, welches endständige Basenpaar die erste DNA trägt, wobei entsprechend dem erfindungsgemäßen Konzept die erste DNA unverändert zurückerhalten werden kann.

Gemäß einer weiteren Variante des Verfahrens bzw. der Oligonucleotide der Erfindung können an definierten Positionen des Oligonucleotids Ribonucleotide eingebaut werden. Erfindungsgemäß können die Restriktionsendonuclease-spezifischen kohäsiven Enden auch ohne Verwendung der Restriktionsendonuclease eingeführt werden. Dies wird an den folgenden Beispielen 9 und 13 erläutert.

**Beispiel 9**

Kohäsives Ende für Eco RI:

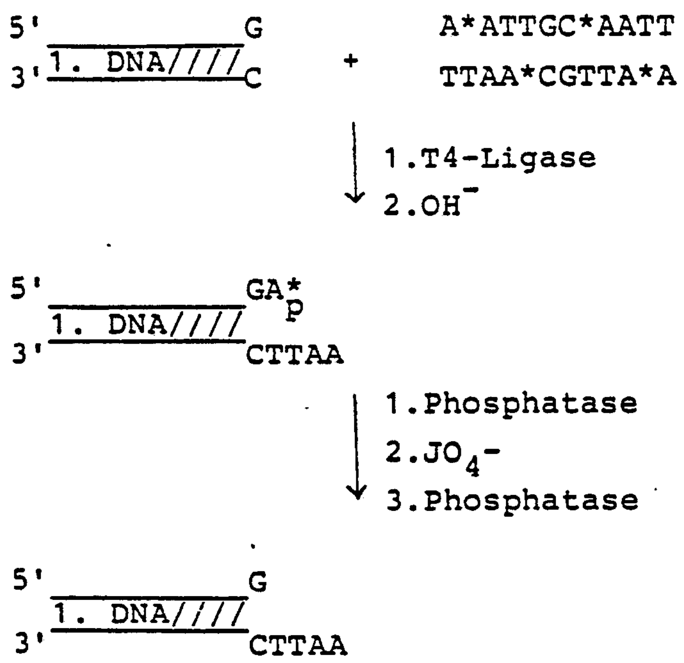

**Beispiel 10**

Kohäsives Ende für Pst I:

5' C
1. DNA////
3' G

\+

TGCAG*CTGCA*
A*CGTCG*ACGT

1.T4-Ligase
2. $OH^-$

5' CTGCAG*
p
1. DNA////
3' G

1.Phosphatase
2.$JO_4^-$
3.Phosphatase

5' CTGCA
1. DNA////
3' G

In den Beispielen 9 und 10 werden, ebenso wie in der folgenden Beschreibung, Ribonucleotide durch * gekennzeichnet.

Durch den Einbau eines Ribonucleotids wird innerhalb der DNA eine definierte alkalilabile Position geschaffen, wie sich aus dem folgenden Reaktionsschema ergibt:

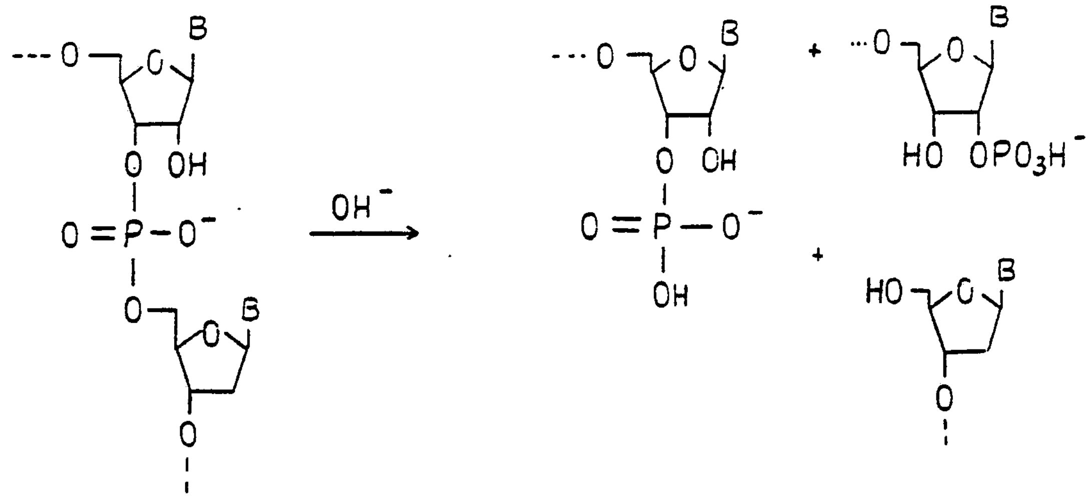


Dabei verbleibt der Phosphatrest der Internucleotidbindung am Ribonucleotid. Nach Behandlung mit Phosphatase wird eine 2', 3'-cis-Diolgruppierung geschaffen, die durch Perjodatbehandlung in bekannter Weise gespalten und ebenfalls in bekannter Weise unter milden alkalischen Bedingungen zur Eliminierung des Ribonucleotids führt. Zum Schluß wird durch erneute Phosphatasebehandlung das 3'-terminale Desoxyribonucleotid freigesetzt. Es kann vorteilhaft sein, diesen Schritt erst später in einer Reaktionssequenz durchzuführen, um dieses kohäsive Ende durch den 3'-Phosphatrest "geschützt" zu behalten.

Ein weiterer Vorteil dieser Verfahrensvariante liegt darin, daß die Ribonucleotidpositionen so gewählt werden können, daß jeweils nur einer der beiden DNA-Stränge eine definierte alkalilabile Bruchstelle erhalten, bzw. der jeweils andere Strang durch die entsprechende Restriktionsendonuclease geschnitten werden kann. Dies wird im folgenden an zwei Beispielen erläutert:

**Beispiel 11**

```
                 OH⁻ spaltbar
                 ↓
5' ____________G A*ATTCGAATT
    1. DNA////
3' ____________C T TAAGCTTAA*
                     ↑
                enzymatisch spaltbar
```

**Beispiel 12**

```
                  enzymatisch spaltbar
                 ↙
5' ____________G AATTCG*AATT
    1. DNA////
3' ____________C TTAA*GCTTAA
                     ↑
                  OH⁻-spaltbar
```

Das Verfahren der Erfindung ermöglicht auch die Einführung von kohäsiven Enden, die nicht selbst komplementär sind. In diesem Falle ist $R^1$ eine nicht in sich selbst komplementäre Tetra- oder Pentanucleotidsequenz oder eine in sich selbst oder nicht in sich selbst komplementäre Hexanucleotidsequenz der Formel V, VI bzw. VII gemäß dem Hauptanspruch (mit den dort angegebenen weiteren Definitionen); $R^2$ ist stets eine zu $R^1$ spiegelbildlich komplementäre Sequenz gleicher Länge mit 4, 5 oder 6 Nucleotiden. Wie weiter oben ausgeführt wurde, weist $R^1$ und/oder $R^2$ ein 5'- oder 3'-endständiges Ribonucleotid auf, wobei gegebenenfalls in dem Spacer $(X)_n$ ein weiteres Ribonucleotid vorzusehen ist.

In Abhängigkeit davon, wo die Ribonucleotide positioniert sind, kann - bei gleicher Nucleotidsequenz - die Länge des kohäsiven Endes beeinflußt werden und das kohäsive Ende entweder an das 3'- oder 5'-Ende der ersten DNA (Fremd-DNA) dirigiert werden.

Die folgenden Beispiele erläutern diese Verfahrensvariante:

**Beispiel 13**

```
5' ____________                C*ACAGC*TGTG
    1. DNA////          +
3' ____________                GTGTC*GACAC*

                        |  1. T4-Ligase
                        |  2. Phosphatase
                        |  3. Perjodat
                        ↓  4. Phosphatase

5' ____________
    1. DNA////
3' ____________GTGT
```

**Beispiel 14**

5' 1. DNA/// 3' + C*ACAG*CTGTG / GTGTCG*ACAC*

↓ 1. T4-Ligase
2. Phosphatase
3. Perjodat
4. Phosphatase

5' 1. DNA/// 3' GTGTC

**Beispiel 15**

5' 1. DNA/// 3' + CACAG*CTGTG* / *GTGTCG*ACAC

↓ 1. T4-Ligase
2. Phosphatase
3. Perjodat
4. Phosphatase

5' CACA 1. DNA/// 3'

**Beispiel 16**

5' 1. DNA/// 3' + CACAGC*TGTG* / *GTGT*CGACAC

↓ 1. T4-Ligase
2. Phosphatase
3. Perjodat
4. Phosphatase

5' CACAG 1. DNA/// 3'

Durch geeignete Positionierung der Ribonucleotide ist auch bei dieser Verfahrensvariante möglich, die alkalilabile Bruchstelle in jeweils nur einem der beiden DNA-Stänge einzuführen. Dies ermöglicht z. B. bei ringförmiger, doppelsträngiger, entweder die Isolierung des Minus-oder des Plus-Stranges.

Das folgende Beispiel verdeutlicht, daß mit Oligonucleotiden I, in denen $R^1$ und $R^2$ in sich selbst oder nicht in sich selbst komplementäre Hexanucleotidsequenzen sind, kohäsive Enden erzeugt werden können, die, in Abhängigkeit von der Positionierung von Ribonucleotiden in den Resten $R^1$, $R^2$ bzw. X, Kettenlängen von 1 - 6 Nucleotiden aufweisen.

**Beispiel 17:**

(Fremd-DNA) C*ACACAGC*TGTGTG / GTGTGTC*GACACAC* $\xrightarrow{OH^-}$ (Fremd-DNA) GTGTGT

(Fremd-DNA) C*ACACAGCT*GTGTG / GTGTGT*CGACACAC* $\xrightarrow{OH^-}$ (Fremd-DNA) GTGTG

(Fremd-DNA) C*ACACAGCTG*TGTG / GTGTG*TCGACACAC* $\xrightarrow{OH^-}$ (Fremd-DNA) GTGT

etc.

(Fremd-DNA) *CACACAGCTGTGT*G / GT*GTGTCGACACAC* $\xrightarrow{OH^-}$ (Fremd-DNA) G

Die vorstehenden Beispiele offenbaren die große Flexibilität des Verfahrens der Erfindung, das immer dann von Vorteil ist, wenn DNA-Fragmente irreversibel miteinander verkoppelt werden sollen oder wenn sich die Anwendung von Restriktionsendonucleasen verbietet, weil das Vorhandensein interner Signalsequenzen sehr wahrscheinlich ist. Dies ist bei hochmolekularen DNA-Fragmenten, z.B. zur Etablierung einer Genbank, fast immer der Fall.

Der in dem Oligonucleotid der Formel I enthaltene Spacer $(X)_n$ stellt kurze Oligonucleotidsequenzen dar, die als notwendiger Spacer unter Berücksichtigung der für die Restriktionsendonuclease-Erkennungsregion notwendigen Nucleotide und der erforderlichen Stabilität der durch Selbstkomplementarität sich bildenden doppelsträngigen DNA ausgewählt wird. Wird die zentrale Tetranucleotidsequenz nur durch die wenig Stabilität vermittelnden AT-Basenpaare gebildet, wie z.B. beim Eco RI-Linker

d(AATTCGAATT)

so kann die Stabilität z.B. durch Verlängerung des Spacers erhöht werden, z. B.

d(AATTCGCGAATT).

Dadurch kann eine weitere Erkennungsregion für eine Restriktionsendonuclease eingeführt werden, z. B. für X = CCCGGG hat der Eco RI-Linker die Formel

Xmal Smal
d (AATTCCCGGGAATT)

Hier wird nicht nur mit dem Linker die Eco RI-Signalsequenz in eine Fremd-DNA eingeführt, die GC als endständiges Basenpaar besitzt, sondern auch noch die recht seltene Erkennungssequenz für Smal/Xmal.

Das folgende Ausführungsbeispiel betrifft die Herstellung und erfindungsgemäße Anwendung eines Oligonucleotids der Erfindung der Formel d(GATCCGGATC).

**Beispiel 18**

Herstellung von d(GATCCGGATC) (vgl. Tetrahedron Let. 1983, 747)

100 mg C-Träger (10 μMol N-benzoyliertes C, kovalent gebunden an "Controlled Pore Glass" (CPG), werden nacheinander mit je 100 μMol der durch MSNT (1,5 Äquivalente/PO⁻) aktivierten N-geschützten, 5'-dimethoxytritylierten Desoxynucleosid-3'-(2-chlorphenyl)-phosphodiester T, A, G, G, C, C, T, A und G umgesetzt. Ein Reaktionszyklus umfaßt Kondensation (45') in Pyridin, Waschen mit Pyridin, Capping mit Essigsäureanhydrid/Pyridin (10'), Waschen mit Pyridin, $CH_2Cl_2$/MeOH (7 : 3) und Abspalten der Dimethoxytritylgruppe mit gesättigtem $ZnBr_2$ in $CH_2Cl_2$/Methanol (7 : 3) für 10' und Waschen mit $CH_2Cl_2$/MeOH (7 : 3) sowie Pyridin.

Die durchschnittlichen Kondensationsausbeuten liegen bei 90 - 95 %.

Nach der letzten Kondensation erfolgt Abspaltung der 2-Chlor-Phenylgruppe durch Behandeln mit Oximatreagenz (vgl. Tetrahedron Let. 1978, 2727),

3 ml, für 24 h, Abspaltung vom Träger und Entfernung der N-Arylgruppen durch Behandlung mit konzentriertem wässrigem Ammoniak, 12 h bei 50°C. Nach dem Einengen und 5-facher Extraktion mit je 5 ml $CHCl_3$ wird die wässrige Lösung durch Anionaustauschchromatographie an DEAE-Cellulose in der üblichen Weise gereinigt (vgl. Liebigs Ann. 1978, 839 und Tetrahedron Let. 1983, 747).

Das gereinigte Material wird in üblicher Weise durch Polyacrylamidgelelektrophorese (20 %, 7M Harnstoff) und Vergleich mit einem Homo-oligo-dT-Kettenlängenstandard und durch Sequenzierung entweder nach Maxam-Gilbert oder nach der "mobility shift"-Methode charakterisiert.

**Ligation:**

Um das Ligationssubstrat zu erhalten, wird pBR 322 mit Bal I (TGG↓CCA) in üblicher Weise linearisiert und auf diese Weise die DNS mit dem stumpfen $^{G}{}_{C}$-Ende versehen. Die Ligation erfolgt in 66 mM Tris-HCl (pH 7,6), 6,6 mM $MgCl_2$, 10 mM Dithiothreitol, 0,4 mM ATP und 4 Einheiten T4-Ligase pro 20 µl Reaktionsvolumen. Der Linker wird im 50-fachen Überschuß über die Termini verwendet. Die Ligationsdauer beträgt 24 h bei 10°C.

Anschließend wird mit Bam HI in der üblichen Weise inkubiert; die entstandenen Bam HI-kohäsiven Enden werden in gleicher Weise wie oben angegeben, jedoch mit lediglich 0,02 Einheiten T4-Ligase, pro 20 µl ligiert. Die Reaktionsdauer beträgt 1 h bei 22°C.

Die Agarosegelelektrophorese zeigt Umwandlung der linearen pB R 322-DNS in circuläre DNS unter Ligationsbedingungen, unter denen nur kohäsive, nicht jedoch stumpfe Enden ligiert werden.

**Tabelle I**

| | | | | | | |
|---|---|---|---|---|---|---|
| TTTT | AAAT | CCCT | GGGT | TCAT | TCGT | TACT |
| TTTA | AAAA | CCCA | GGGA | TCAA | | TACA |
| TTTC | AAAC | CCCC | GGGC | TCAC | TCGT | TACC |
| TTTG | AAAG | CCCG | GGGG | TCAG | TCGG | TACG |
| TTCT | | CCTT | GGTT | TGCT | TGAT | TAGT |
| TTCA | AATA | CCTA | GGTA | | TGAA | TAGA |
| TTCC | AATC | CCTC | GGTC | TGCC | TGAC | TAGC |
| TTCG | AATG | CCTG | GGTG | TGCG | TGAG | TAGG |
| TTAT | AACT | CCAT | GGAT | CTAT | CTGT | GATT |
| | AACA | CCAA | GGAA | CTAA | CTGA | GATA |
| TTAC | AACC | CCAC | GGAC | CTAC | CTGC | |
| TTAG | AACG | CCAG | GGAG | | CTGG | GATG |
| TTGT | AAGT | CCGT | GGCT | CATT | CAGT | GACT |
| TTGA | AAGA | CCGA | GGCA | CATA | CAGA | GACA |
| TTGC | AAGC | CCGC | | CATC | CAGC | GACC |
| TTGG | AAGG | | GGCG | | CAGG | GACG |
| TATT | | CTCT | GTGT | CGTT | CGAT | |
| | ATAA | CTCA | GTGA | CGTA | CGAA | |

| | | | | | |
|---|---|---|---|---|---|
| TATC | ATAC | CTCC | GTGC | CGTC | CGAC |
| TATG | ATAG | CTCG | GTGG | CGTG | CGAG |
| | | | | | |
| TCTT | ACAT | CACT | GAGT | ATCT | ATGT |
| TCTA | ACAA | CACA | GAGA | ATCA | ATGA |
| TCTC | ACAC | CACC | GAGC | ATCC | ATGC |
| TCTG | ACAG | CACG | GAGG | ATCG | ATGG |
| | | | | | |
| TGTT | AGAT | CGCT | GCGT | ACTT | |
| TGTA | AGAA | CGCA | GCGA | ACTA | ACGA |
| TGTC | AGAC | CGCC | | ACTC | ACGC |
| TGTG | AGAG | | GCGG | ACTG | ACGG |
| | | | | | |
| TAAT | ATTT | CTTT | GTTT | AGTT | |
| TAAA | ATTA | CTTA | GTTA | AGTA | AGCA |
| TAAC | ATTC | CTTC | GTTC | AGTC | AGCC |
| TAAG | ATTG | CTTG | GTTG | AGTG | AGCG |
| | | | | | |
| TCCT | ACCT | CAAT | GAAT | GTCT | GTAT |
| TCCA | ACCA | CAAA | GAAA | GTCA | GTAA |
| TCCC | ACCC | CAAC | GAAC | GTCC | |
| TCCG | ACCG | CAAG | GAAG | GTCG | GTAG |
| | | | | | |
| TGGT | AGGT | CGGT | GCCT | GCTT | GCAT |
| TGGA | AGGA | CGGA | GCCA | GCTA | GCAA |
| TGGC | AGGC | CGGC | GCCC | GCTC | GCAC |
| TGGG | AGGG | CGGG | GCCG | GCTG | GCAG |

**Patentansprüche**

1. Verfahren zur definierten Verknüpfung doppelhelikaler DNA-Fragmente über kohäsive Enden mit den Schritten:

1. Umsetzung eines ersten DNA-Fragmentes an dessen stumpfen Ende mit einer kurzen, beidseitig stumpfen doppelhelikalen Linker-DNA in Gegenwart von Ligasen,
2. Spaltung der so erhaltenen DNA im Bereich der Linkersequenz unter Ausbildung des eine Sequenz von 1 - 6 Nucleotide aufweisenden kohäsiven Endes und
3. Verknüpfung der so erhaltenen, das kohäsive Ende aufweisenden DNA mit einem ein kohäsives Ende aufweisenden zweiten DNA-Fragment in Gegenwart von Ligasen,

wobei die kohäsiven Enden der ersten und zweiten DNA-Fragmente zueinander komplementär sind, dadurch gekennzeichnet, daß man als Linker-DNA ein in sich selbst komplementäres, unter Ligationsbedingungen als Doppelhelix vorliegendes Oligonucleotid der allgemeinen Formel I

$$(5') R^1 - R^2 - (X)_n - R^2 (3') \qquad (I)$$

in der $R^1$ und $R^2$ von links nach rechts gelesen identische, in sich selbst komplementäre Tetradesoxy- oder Monoribotridesoxynucleotidsequenzen aus der Gruppe der Sequenzen der Formel II

(5')A*ATT*(3')
T*TAA*
A*TAT*
T*ATA*
G*GCC*
C*CGG*
G*CGC*
C*GCG*
G*ATC*
G*TAC*
C*ATG*
C*TAG*
A*CGT*
A*GCT*
T*CGA*
T*GCA*

(II)

in denen A, C, G und T bzw. A*, C*, G* und T* Adenin-, Cytosin-, Guanin bzw. Thymidinnucleotide sind, wobei A*, C*, G* und T* entweder das 5' bzw. 3'-endständige Desoxy- oder das 5' bzw. 3'-endständige Ribonucleotid bedeuten und das Ribothymidinnucleotid T* durch das Ribouridinnucleotid ersetzt sein kann,

oder

$R^1$ eine Pentadesoxy- oder Monoribotetradesoxynucleotidsequenz aus der Gruppe der Sequenzen der Formel III

(5')A*ANTT*(3')
T*TNAA*
A*TNAT*
T*ANTA*
G*GNCC*
C*CNGG*
G*CNGC*
C*GNCG*
G*ANTC*
G*TNAC*
C*ANTG*
C*TNAG*
A*CNGT*
A*GNCT*
T*CNGA*
T*GNCA*

(III)

bedeutet, wobei A, C, G und T bzw. A*, C*, G* und T* wie oben definiert sind und N eines der Nucleotide A, T, C oder G bedeutet,

und $R^2$ eine Pentadesoxy- oder Monoribotetradesoxynucleotidsequenz aus der Gruppe der Sequenzen der Formel IV

(5')A*AN'TT*(3')
T*TN'AA*
A*TN'AT*
T*AN'TA*
G*GN'CC*
C*CN'GG*
G*CN'GC*
C*GN'CG*
G*AN'TC*
G*TN'AC*
C*AN'TG*
C*TN'AG*
A*CN'GT*
A*GN'CT*
T*CN'GA*
T*GN'CA*

(IV)

bedeuten, wobei A, C, G und T bzw. A*, C*, G* und T* wie oben definiert sind, N' das zu N komplementäre Nucleotid A, T, C oder G ist und die Sequenzen der Gruppen $R^1$ und $R^2$ bis auf die zueinander komplementären Nucleotide N und N', von links nach rechts gelesen, identisch sind,

oder
$R^1$ eine der durch Kombination der vier Nucleotide A, C, G und T möglichen, nicht in sich selbst komplementären Tetra- oder Pentanucleotidsequenzen der Formeln V oder VI oder eine in sich selbst komplementäre oder nicht in sich selbst komplementäre Hexanucleotidsequenz der Formel VII

$$(5')N^1\text{-}N^2\text{-}N^3\text{-}N^4(3') \quad (V)$$

$$(5')N^1\text{-}N^2\text{-}N^3\text{-}N^4\text{-}N^5(3') \quad (VI)$$

$$(5')N^1\text{-}N^2\text{-}N^3\text{-}N^4\text{-}N^5\text{-}N^6(3') \quad (VII)$$

bedeuten,
wobei $N^1$, $N^2$, $N^3$, $N^4$, $N^5$ und $N^6$ eins der Nucleotide A, T, C oder G ist, und $R^2$ eine zu $R^1$ spiegelbildlich komplementäre Tetra-, Penta- bzw. Hexanucleotidsequenz der Formeln V, VI bzw. VII mit den oben angegebenen Bedeutungen für $N^1$ , $N^2$, $N^3$, $N^4$, $N^5$ und $N^6$ ist und entweder $R^1$ eine Monoribotridesoxy-, Monoribotetradesoxy- oder Monoribopentadesoxynucleotidsequenz und $R^2$ eine Tetradesoxy-, Pentadesoxy- oder Hexadesoxynucleotidsequenz oder $R^1$ eine Tetradesoxy-, Pentadesoxy- oder Hexadesoxynucleotidsequenz und $R^2$ eine Monoribotridesoxy-, Monoribotetradesoxy- oder Monoribopentadesoxynucleotidsequenz ist, oder $R^1$ eine zu $R^2$ spiegelbildlich komplementäre Monoribotridesoxy-, Monoribotetradesoxy- oder Monoribopentadesoxysequenz ist, wobei sich das Ribonucleotid am 5'- oder 3'-Ende von $R^1$ oder am 5' oder 3'-Ende von $R^2$ befindet, und wobei 5'- bzw. 3'-ständiges Ribothymidin durch Ribouridin ersetzt sein kann,
X ein Oligonucleotid aus der Gruppe der Sequenzen der Formel VIII

(5')C*G*(3')
G*C*
T*A*
A*T*
G*CGG*
G*GCC*
A*ATT*
T*TAA*
C*CCGGG*
G*GGCCC*
A*AATTT*
T*TTAAA*

(VIII)

in denen A, C, G und T bzw. A*, C*, G* und T* wie oben definiert sind, und
n eine ganze Zahl von 1 - 5 bedeutet, jedoch mit den Ausnahmen

1.) wenn $R^1$ = R = CATG kann X nur AT, TA, GC oder CG bedeuten und
2.) wenn $R^1$ = $R^2$ = CCGG kann X nur CG, GC, AATT oder TTAA bedeuten,

verwendet,
und die Spaltung der so erhaltenen doppelhelikalen DNA im Linkerbereich bei Nucleotidsequenzen, die keine Ribonucleotide enthalten, in an sich bekannter Weise mit einer für das zu erzeugende kohäsive Ende spezischen Restriktionsendonuclease der Klassen II oder III und bei Ribonucleotide enthaltenden Nucleotidsequenzen mit Basen oder Ribonucleasen vornimmt.

2. Verfahren nach Anspruch 1, dadurch gekennzeichnet, daß man das ein kohäsives Ende aufweisende erste DNA-Fragment vor der Verknüpfung mit dem zweiten DNA-Fragment mit einer an sich bekannten, kohasive Enden unterschiedlicher Restriktionsspezifität aufweisenden Adapter-DNA umsetzt.

3. Unter Ligationsbedingungen doppelhelikale Linker-DNA der Formel I

$$(5')R^1 - (X)_n - R^2(3') \quad (I),$$

in der $R^1$, $R^2$, X und n wie oben definiert sind, jedoch mit den Ausnahmen

1.) wenn $R^1$ = $R^2$ = CATG kann X nur AT, TA, GC oder CG bedeuten und
2.) wenn $R^1$ = $R^2$ = CGGG kann X nur CG, GC, AATT oder TTAA bedeuten.

**Claims**

1. Process for the defined linkage of double-helical DNA fragments via cohesive ends, with the following steps:

1. Reaction of the blunt end of a first DNA fragment with a short double-helical linker DNA, which is blunt at both ends, in the presence of ligases,
2. Cleavage of the DNA thus obtained in the region of the linker sequence, with the formation of a cohesive end having a sequence of 1 - 6 nucleotides, and
3. Linkage of the DNA thus obtained, which has the cohesive end, with a second DNA fragment having a cohesive end, in the presence of ligases,

the cohesive ends of the first and second DNA fragments being complementary to one another, characterised in that the linker DNA used is a self-complementary oligonucleotide which is in the form of a double helix under ligation conditions and has the general formula I

$$(5')\ R^1 - (X)_n - R^2\ (3') \qquad (I)$$

in which $R^1$ and $R^2$, reading from left to right, are identical self-complementary tetradeoxynucleotide or monoribotrideoxynucleotide sequences from the group of sequences of the formula II

(5')A*ATT*(3')
T*TAA*
A*TAT*
T*ATA*
G*GCC*
C*CGG*
G*CGC* (II)
C*GCG*
G*ATC*
G*TAC*
C*ATG*
C*TAG*
A*CGT*
A*GCT*
T*CGA*
T*GCA*

in which A, C, G and T, and A*, C*, G* and T* are adenine, cytosine, guanine and thymidine nucleotides respectively, A*, C*, G* and T* denoting either the 5'- or 3'-terminal deoxynucleotide or the 5'- or 3'-terminal ribonucleotide, and it being possible for the ribothymidine nucleotide T* to be replaced by the ribouridine nucleotide,

or

$R^1$ denotes a pentadeoxynucleotide or monoribotetradeoxynucleotide sequence from the group of sequences of the formula III

(5')A*ANTT*(3')
T*TNAA*
A*TNAT*
T*ANTA*
G*GNCC*
C*CNGG*
G*CNGC*
C*GNCG*
G*ANTC* (III)
G*TNAC*
C*ANTG*
C*TNAG*
A*CNGT*
A*GNCT*
T*CNGA*
T*GNCA*

A, C, G and T, and A*, C*, G* and T* being defined as above, and N denoting one of the nucleotides A, T, C or G, and $R^2$ denotes a pentadeoxynucleotide or monoribotetradeoxynucleotide sequence from the group of

sequences of the formula IV

(5')A*AN'TT*(3')
T*TN'AA*
A*TN'AT*
T*AN'TA*
G*GN'CC*
C*CN'GG*
G*CN'GC*
C*GN'CG*
G*AN'TC*
G*TN'AC*
C*AN'TG*
C*TN'AG*
A*CN'GT*
A*GN'CT*
T*CN'GA*
T*GN'CA*
(IV)

A, C , G and T, and A*, C*, G* and T* being defined as above, N' being the nucleotide A, T, C or G which is complementary to N, and the sequences of th groups $R^1$ and $R^2$, read from left to right, being identical apart from the nucleotides N and N' which are complementary to one another,

or

$R^1$ denotes one of the tetranucleotide or pentanucleotide sequences possible by combination of the four nucleotides A, C, G and T, which is not self-complementary, of the formulae V or VI, or a hexanucleotide sequence of the formula VII which is self-complementary or is not self-complementary.

(5')$N^1$-$N^2$-$N^3$-$N^4$(3') (V)

(5')$N^1$-$N^2$-$N^3$-$N^4$-$N^5$(3') (VI)

(5')$N^1$-$N^2$-$N^3$-$N^4$-$N^5$-$N^6$(3') (VII)

$N^1$, $N^2$, $N^3$, $N^4$, $N^5$, and $N^6$ being one of the nucleotides A, T, C or G, and $R^2$ being a tetranucleotide, pentanucleotide or hexanucleotide sequence which is reverse complementary to $R^1$, and has the formulae V, VI or VII, with the abovementioned meanings for $N^1$, $N^2$, $N^3$, $N^4$, $N^5$ and $N^6$, and either $R^1$ is a monoribotrideoxynucleotide, monoribotetradeoxynucleotide or monoribopentadeoxynucleotide sequence and $R^2$ is a tetradeoxynucleotide, pentadeoxynucleotide or hexadeoxynucleotide sequence, or $R^1$ is a tetradeoxynucleotide, pentadeoxynucleotide or hexadeoxynucleotide sequence and $R^2$ is a monoribotrideoxynucleotide, monoribotetradeoxynucleotide or monoribopentadeoxynuc leotide sequence, or $R^1$ is a monoribotrideoxynucleotide, monoribotetradeoxynucleotide or monoribopentadeoxynucleotide sequence which is reverse complementary to $R^2$, the ribonucleotide being located at the 5'- or 3'-end of $R^1$ or at the 5'- or 3'-end of $R^2$, and it being possible for 5'- or 3'-ribothymidine to be replaced by ribouridine,

X denotes an oligonucleotide from the group of sequences of the formula VIII

(5')C*G*(3')
G*C*
T*A*
A*T*
G*CGG*
G*GCC*
A*ATT*
T*TAA*
C*CCGGG*
G*GGCCC*
A*AATTT*
T*TTAAA*
(VIII)

in which A, C, G and T, and A*, C*, G* and T* are defined as above, and n denotes an integer from 1 to 5, but with the exceptions

1) if $R^1$ = $R^2$ = CATG X can only mean AT, TA, GC or CC and
2) if $R^1$ = $R^2$ = CCGG X can only mean CG, GC, AATT or TTAA

and the cleavage in the linker region of the double-helical DNA thus obtained is carried out, for nucleotide

sequences which do not contain ribonucleotides, in a manner known per se using a restriction endonuclease of classes II or III which is specific for the cohesive end to be produced, and, for nucleotide sequences containing ribonucleotides, using bases or ribonucleases.

2. Process according to claim 1, characterized in that the first DNA fragment having a cohesive end is, before linkage with the second DNA fragment, reacted with an adapter DNA which is known per se and has cohesive ends of different restriction specificity.

3. Linker DNA which is double-helical under ligation conditions and has the formula I

$$(5')R^1 - (X)_n - R^2(3') \quad (I)$$

in which $R^1$, $R^2$, X and n are as defined above, but with the exceptions

1) if $R^1 = R^2 =$ CATG X can only mean AT, TA, GC or CG and
2) if $R^1 = R^2 =$ CCGG X can only mean CG, GC, AATT or TTAA

## Revendications

1. Procédé pour la liaison définie de fragments d'ADN à double hélice par des extrémités cohésives, comportant les étapes suivantes:

1. réaction d'un premier fragment d'ADN, en ses extrémités tronquées, avec un ADN linker court, à double hélice, tronqué des deux côtés, en présence de ligases,
2. coupure de l'ADN ainsi obtenu dans la région de la séquence du linker, avec formation de l'extrémité cohésive présentant une séquence de 1 - 6 nucléotides, et
3. liaison de l'ADN ainsi obtenu, présentant l'extrémité cohésive, avec un second fragment d'ADN présentant une extrémité cohésive, en présence de ligases,

les extrémités cohésives du premier fragment d'ADN et du second étant complémentaires l'une de l'autre, caractérisé par le fait qu'on utilise en tant qu'ADN linker un oligonucléotide complémentaire en soi, présent sous forme de double hélice dans des conditions de ligature, correspondant à la formule générale I

$$(5')\ R^1 - (X)_n - R^2\ (3') \quad (I)$$

dans laquelle $R^1$ et $R^2$ représentent des séquences tétradésoxy- ou monoribotridésoxynucléotidiques complémentaires en soi, identiques lues de gauche à droite, choisies parmi les séquences de formule II

(5')A*ATT*(3')
T*TAA*
A*TAT*
T*ATA*
G*GCC*
C*CGG*
G*CGC* (II)
C*GCG*
G*ATC*
G*TAC*
C*ATG*
C*TAG*
A*CGT*
A*GCT*
T*CGA*
T*GCA*

dans lesquelles A, C, G et T ou respectivement A*, C*, G* et T* sont des nucléotides à adénine, à cytosine, à guanine ou à thymidine, A*, C*, G* et T* représentant soit le désoxyribonucléotide en terminaison 5' ou 3', soit le ribonucléotide en terminaison 5' ou 3', et le ribonucléotide à thymidine T* pouvant être remplacé par le ribonucléotide à uridine,

ou

$\overline{R^1}$ représente une séquence pentadésoxy- ou monoribotétradésoxynucléotidique choisie parmi les séquences de formule III

(5')A*ANTT*(3')
T*TNAA*
A*TNAT*
T*ANTA*
G*GNCC*
C*CNGG*
G*CNGC*
C*GNCG*
G*ANTC*
G*TNAC*
C*ANTG*
C*TNAG*
A*CNGT*
A*GNCT*
T*CNGA*
T*GNCA*

(III)

dans lesquelles A, C, G et T ou, respectivement, A*, C*, G* et T* sont tels que définis plus haut, et N représente un des nucléotides A, T, C ou G, et

$R^2$ représente une séquence pentadésoxy- ou monoribotétradésoxynucléotidique choisie parmi les séquences de formule IV

(5')A*AN'TT*(3')
T*TN'AA*
A*TN'AT*
T*AN'TA*
G*GN'CC*
C*CN'GG*
G*CN'GC*
C*GN'CG*
G*AN'TC*
G*TN'AC*
C*AN'TG*
C*TN'AG*
A*CN'GT*
A*GN'CT*
T*CN'GA*
T*GN'CA*

(IV)

A, C, G et T ou, respectivement, A*, C*, G* et T* étant tels que définis plus haut, N' étant le nucléotide A, T, C ou G complémentaire de N, et les séquences des groupes $R^1$ et $R^2$ étant identiques, lues de gauche à droite, jusqu'aux nucléotides N et N' complémentaires l'un de l'autre,

<u>ou</u>

$R^1$ représente une des séquences tétra- ou pentanucléotidiques de formules V ou VI, non complémentaires en soi, possibles par combinaison des quatre nucléotides A, C, G et T, ou une séquence hexanucléotidique complémentaire en soi ou non complémentaire en soi, de formule VII

(5')$N^1$-$N^2$-$N^3$-$N^4$(3') (V)

(5')$N^1$-$N^2$-$N^3$-$N^4$-$N^5$(3') (VI)

(5')$N^1$-$N^2$-$N^3$-$N^4$-$N^5$-$N^6$(3') (VII)

$N^1$, $N^2$, $N^3$, $N^4$, $N^5$ et $N^6$ étant un des nucléotides A, T, C, ou G, et $R^2$ étant une séquence tétra-, penta- ou hexanucléotidique complémentaire en tant qu'image de $R^1$ dans un miroir, de formules V, VI ou VII, avec les significations données plus haut pour $N^1$, $N^2$, $N^3$, $N^4$, $N^5$ et $N^6$, et soit $R^1$ étant une séquence monoribotridésoxy-, monoribotétradésoxy- ou monoribopentadésoxynucléotidique, et $R^2$ étant une séquence tétradésoxy-, pentadéesoxy- ou hexadésoxynucléotidique, soit $R^1$ étant une séquence tétradésoxy-, pentadésoxy- ou hexadésoxynucléotidique, et $R^2$ étant une séquence monoribotridésoxy-, monoribotétradésoxy- ou monoribopentadésoxynucléotidique, soit $R^1$ étant une séquence monoribotridésoxy-, monoribotétradésoxy- ou monoribopentadésoxy complémentaire en tant qu'image de $R^2$ dans un miroir, le ribobucléotide se trouvant à l'extrémité 5' ou 3' de $R^1$ ou à l'extrémité 5' ou 3' de $R^2$, et la ribothymidine se trouvant en 5' ou 3' pouvant être remplacée par la ribo-uridine;

X représente un oligonucléotide choisi parmi les sequences de formule VIII

(5')C*G*(3')
G*C*
T*A*
A*T*
G*CGG* (VIII)
G*GCC*
A*ATT*
T*TAA*
C*CCGGG*
G*GGCCC*
A*AATTT*
T*TTAAA*

dans lesquelles A, C, G et T ou, respectivement, A*, C*, G* et T* sont tels que définis plus haut, et n représente un nombre entier valant de 1 à 5, avec toutefois les restrictions suivantes:

1) lorsque $R^1 = R^2$ = CATG, X ne peut représenter que AT, TA, GC ou CG, et
2) lorsque $R^1 = R^2$ = CCGG, X ne peut représenter que CG, GC, AATT ou TTAA,

et on effectue la coupure de l'ADN à double hélice ainsi obtenu, dans la région du linker, dans le cas de séquences nucléotidiques qui ne contiennent pas de ribonucléotides, d'une façon connue en soi, avec une endonucléase de restriction de la classe II ou III, spécifique pour l'extrémité cohésive à produire, et dans le cas de séquences nucléotidiques contenant des ribonucléotides, avec des bases ou des ribonucléases.

2. Procédé selon la revendication 1, caractérisé par le fait que l'on fait réagir un premier fragment d'ADN présentant une extrémité cohésive, avant la liaison avec le second fragment d'ADN, avec un ADN de raccordement (ADN adapter) connu en soi, présentant une spécificité de restriction distincte des extrémités cohésives.

3. ADN linker à double hélice dans des conditions de oligature, de formule I

$$(5')\ R^1 - (X)_n - R^2\ (3') \qquad (2)$$

dans laquelle $R^1$, $R^2$, X et n sont tels que définis plus haut, avec toutefois les restrictions suivantes:

1) lorsque $R^1 = R^2$ = CATG, X ne peut représenter que AT, TA, GC ou CG, et
2) lorsque $R^1 = R^2$ = CCGG, X ne peut représenter que CG, GC, AATT ou TTAA.

SCHEMA 1

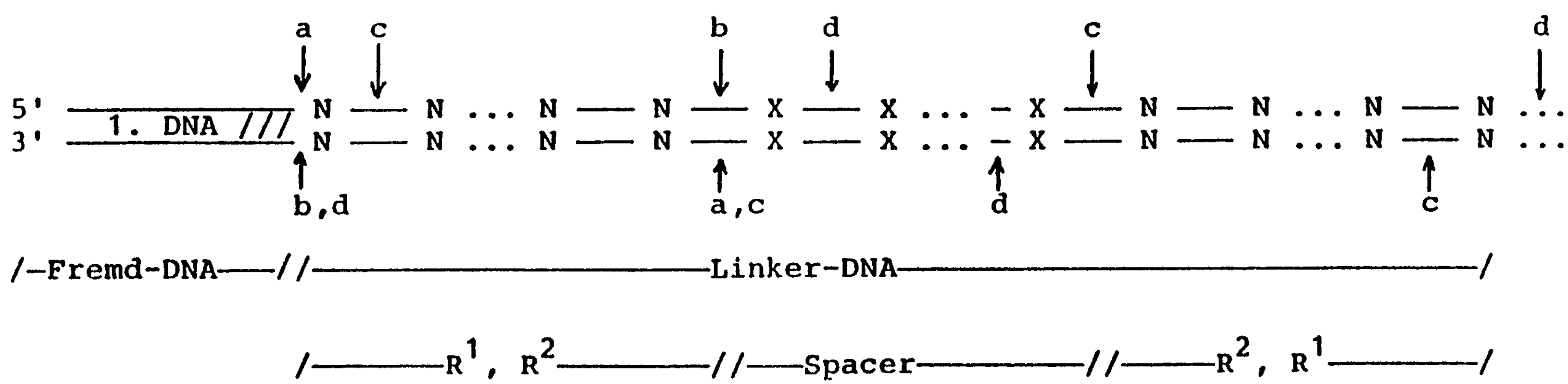
a
c
b
d
c
d
5'
3'
1. DNA ///
N
X
b,d
a,c
d
c
/–Fremd-DNA—//—Linker-DNA—/
/—R1, R2—//—Spacer—//—R2, R1—/